(19) Europäisches Patentamt
European Patent Office
Office européen des brevets

(11) EP 4 603 513 A1

(12) EUROPEAN PATENT APPLICATION
published in accordance with Art. 153(4) EPC

(43) Date of publication:
20.08.2025 Bulletin 2025/34

(51) International Patent Classification (IPC):
C07K 19/00 (2006.01)        C12N 15/85 (2006.01)
A61K 38/20 (2006.01)        A61K 39/00 (2006.01)

(21) Application number: 22961954.9

(22) Date of filing: 23.12.2022

(52) Cooperative Patent Classification (CPC):
Y02A 50/30

(86) International application number:
PCT/CN2022/141443

(87) International publication number:
WO 2024/077776 (18.04.2024 Gazette 2024/16)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 13.10.2022 CN 202211255481

(71) Applicant: Shenzhen Baishitong Technology
Development Company
Limited
Shenzhen, Guangdong 518051 (CN)

(72) Inventors:
• LOU, Jing
  Shenzhen, Guangdong 518051 (CN)
• CHEN, Jianhe
  Shenzhen, Guangdong 518051 (CN)
• SU, Dongmei
  Shenzhen, Guangdong 518051 (CN)
• JIN, Zheng
  Shenzhen, Guangdong 518051 (CN)
• LV, Yunying
  Shenzhen, Guangdong 518051 (CN)

• ZHANG, Ruolan
  Shenzhen, Guangdong 518051 (CN)
• PEI, Ruochen
  Shenzhen, Guangdong 518051 (CN)
• OU, Yanmei
  Shenzhen, Guangdong 518051 (CN)
• QU, Xiao
  Shenzhen, Guangdong 518051 (CN)
• XIE, Xie
  Shenzhen, Guangdong 518051 (CN)
• ZHANG, Jing
  Shenzhen, Guangdong 518051 (CN)
• ZENG, Lin
  Shenzhen, Guangdong 518051 (CN)

(74) Representative: Kailuweit & Uhlemann
Patentanwälte
Partnerschaft mbB
Bamberger Straße 49
01187 Dresden (DE)

Remarks:
The complete document including Reference
Table(s) and the Sequence Listing(s) can be
downloaded from the EPO website

(54) **USE OF ANTIBODY MUTATION METHOD FOR THERAPEUTIC ANTIBODY DRUG**

(57) The present disclosure provides a bifunctional molecule formed by connecting an Fc-mutated antibody to a cytokine, and use of the bifunctional molecule in the preparation of a therapeutic antibody drug. These Fc-mutated monoclonal antibodies or monoclonal antibody/antigen complexes or Fc fusion proteins can significantly reduce *in vivo* toxic side effects while maintaining their pre-mutation *in vivo* and *in vitro* biological activity.

Proliferation of CTLL2 Cell

| | IL2-Fcwt | IL2-Fcmu |
|---|---|---|
| EC50 | 0.9435 | 0.9101 |

FIG.1

**Description**

**FIELD OF TECHNOLOGY**

The present disclosure belongs to the field of pharmaceutical technology, and particularly relates to a use of an antibody mutation for a therapeutic antibody drug.

**BACKGROUND**

The Fc fusion protein is a novel type of recombinant protein, which binds the Fc fragment (fragment crystallizable) of an immunoglobulin to a functional protein molecule with biological activity by using genetic engineering technology to make the functional protein molecule possess antibody-like properties . Monoclonal antibodies/antibody Fc fusion proteins have been widely used in clinical and have achieved great success. As of September 2014, nine human IgG-Fc fusion protein drugs have been approved by the U.S. Food and Drug Administration (FDA) for clinical use.

Although many monoclonal antibodies/antibody fusion proteins under research exhibit excellent efficacy, certain toxic side effects emerge, such as liver and lung damage, which may lead to the failure or stagnation of clinical research under severe cases.

SUMMARY

In view of the above reasons, the object of the present disclosure is to provide a use of an antibody mutation for a therapeutic antibody drug.

In order to achieve the objective of the present disclosure, the technical solution of the present disclosure is as follows:

The present disclosure provides a bifunctional molecule, comprising an Fc-mutated antibody and a cytokine, wherein the Fc-mutated antibody and the cytokine are connected with each other.

In particular, an Fc mutation in the Fc-mutated antibody is selected from:
H310A/H435Q, I253A, S254A, R255A, K288A, L309A, H310A, S415A, H433A, H435A, H435R, Y436A, H310Q/H433N, M252Y/T256Q, and M252F/T256D.

In particular, the antibody is selected from:
IgG1, IgG4, HER2, HER3, EGFR, PDL1, CD19, CD20, CD22, CD24, CD33, CD40, CD40L, CD73, CD276, VEGFR, TIGIT, TIM3, LAG3, CXCR3, CXCR5, CCR3, CCR4, CCR9, and PD1.

In particular, the cytokine is selected from:
IL2 and mutants thereof, IL7, IL12 and mutants thereof, IL15, IL18, IL21, IL2-CD25, Neo 2/15, IFNα, IFNα2b and mutants thereof, IFNγ, TNFα, GM-CSF, FLt3, and CCL21.

The present disclosure provides a method for amplifying the bifunctional molecule, comprising connecting a cytokine to an Fc-mutated antibody to form a fusion protein; constructing the fusion protein into an expression vector; transfecting the expression vector into cells, and performing purification.

The present disclosure provides a therapeutic antibody drug, comprising the bifunctional molecule.

The present disclosure further provides a use of the bifunctional molecule in the preparation of a drug.

In particular, the drug is a therapeutic antibody drug.

Compared with the prior art, the bifunctional molecule provided by the present disclosure, which comprises a mutated monoclonal antibody, a monoclonal antibody/antigen complex, or an Fc fusion protein, indicates through *in vivo* and *in vitro* studies that the Fc-mutated monoclonal antibody, monoclonal antibody/antigen complex, or Fc fusion protein can significantly reduce *in vivo* toxic side effects while maintaining their pre-mutation *in vivo* and *in vitro* biological activity, demonstrating good application prospects.

**BRIEF DESCRIPTION OF THE DRAWINGS**

The present disclosure is further described below with reference to the accompanying drawings and embodiments.

FIG. 1 shows the *in vitro* biological activity of IL2-Fcmu.

FIG. 2 shows the *in vivo* antitumor activity of IL2-Fcmu.

FIG. 3 shows the *in vivo* antitumor activity of mIL12sc-Fcmu.

FIG. 4 shows the *in vitro* biological activity of hIL15sc -Fcmu.

FIG. 5 shows the *in vivo* antitumor activity of hIL15sc -Fcmu.

FIG. 6 shows the *in vitro* biological activity of a human IL2/hu22F8mu complex.

FIG. 7 shows the *in vivo* antitumor activity of a human IL2/hu22F8mu complex.

FIG. 8 shows the *in vitro* biological activity of a bifunctional molecule hu147mu-hEL15sc.

FIG. 9 shows the *in vivo* antitumor activity of a bifunctional molecule hu147mu-hEL15sc.

FIG. 10 shows the *in vitro* biological activity of a bifunctional molecule hu3E10mu-hIL15sc .

FIG. 11 shows the *in vivo* antitumor activity of a bifunctional molecule hu3E10mu-hIL15sc.

FIG. 12 shows the *in vitro* biological activity of bifunctional molecule hu19H6mu-IL15sc.

FIG. 13 shows the *in vivo* antitumor activity of a bifunctional molecule hu19H6mu-hIL15sc.

FIG. 14 shows the *in vitro* biological activity of bifunctional molecule hu609mu-IL15sc.

FIG. 15 shows the *in vivo* antitumor activity of bifunctional molecule hu609mu-IL15.

FIG. 16 shows the *in vitro* biological activity of bifunctional molecule ch158mu-IL15sc.

FIG. 17 shows the *in vivo* antitumor activity of a bifunctional molecule ch158mu-IL15sc.

## DETAILED DESCRIPTION

Our research has demonstrated that:

the liver and lung damage mentioned above is related to the presence of the monoclonal antibodies/antibody Fc fusion proteins in the liver and lungs;

once some highly active monoclonal antibodies/antibody Fc fusion proteins are present in the liver and lungs, they can induce inflammatory responses in the liver and lungs, thus leading to toxic side effects.

the presence of these monoclonal antibodies/antibody Fc fusion proteins in the liver and lungs is related to Fc;

Fc binds to FcRn (an IgG antibody receptor located on the cell membrane) that is abundant in liver and lung tissues, causing the presence of these monoclonal antibodies/antibody Fc fusion proteins in the liver and lungs;

reducing the binding of Fc to FcRn can effectively alleviate the toxicity of monoclonal antibodies/antibody Fc fusion proteins to the liver and lungs;

reducing the binding of Fc to FcRn does not affect or affects the *in vivo* and *in vitro* biological activity of monoclonal antibodies/antibody Fc fusion proteins in an acceptable level.

The present disclosure will be further described in detail below with reference to specific embodiments, and the following embodiments are not intended to limit the present disclosure, but are merely used to illustrate the present disclosure. The experimental methods used in the following embodiments, unless otherwise specified, are conducted under conventional conditions. Materials, reagents, etc., used in the following embodiments, unless otherwise specified, are commercial available.

**Embodiment 1 Use of Antibody Mutation Technology in IL2-Fc Fusion Protein**

This embodiment takes a fusion protein formed by human IL2 (Proleukin, amino acid sequence as shown in SEQ ID NO: 2) and the mutated human IgG4 Fc as an example to demonstrate that the Fc-mutated fusion protein IL2-Fcmu greatly reduces the *in vivo* toxicity while maintaining the *in vivo* and *in vitro* biological activity.

The Fc mutation technology, when applied to other types of IL2-Fc fusion proteins, can achieve the same effect. Examples include the wild-type IL2 (amino acid sequence as shown in SEQ ID NO: 1), Mutein (amino acid sequence as shown in SEQ ID NO: 3), Superkine (amino acid sequence as shown in SEQ ID NO: 4), etc.

## 1.1 Preparation of Fusion Protein IL2-Fcwt and Mutated Fusion Protein IL2-Fcmu

Human IL2 (Proleukin, amino acid sequence as shown in SEQ ID NO:2) was linked to human IgG4-Fc (amino acid sequence as shown in SEQ ID NO: 16) to form the IL2-Fcwt fusion protein (amino acid sequence as shown in SEQ ID NO: 18). Human IL2 was linked to the mutated human IgG4 Fc (amino acid sequence as shown in SEQ ID NO: 17) to form the IL2-Fcmu fusion protein (amino acid sequence as shown in SEQ ID NO: 19). The genes for IL2-Fcwt and IL2-Fcmu were each constructed into the pcDNA3.4 expression vector, followed by transfected into Expi-293F cells, and purification was performed using Protein G to obtain the fusion proteins IL2-Fcwt and IL2-Fcmu, with a purity of >95%. The fusion proteins were then quantified, aliquoted, and stored at -80°C for later use.

## 1.2 *In Vitro* Biological Activity of IL2-Fcmu

This embodiment demonstrates the biological activity of IL2-Fcmu and IL2-Fcwt through a CTLL2 cell proliferation assay. The method was as follows: CTLL2 cells were diluted to $5 \times 10^4$ cells/ml using 1640 culture medium containing 10% FBS, and 100 $\mu$l of the cell suspension was added to each well of a cell culture plate. IL2-Fcwt and IL2-Fcmu were diluted to 100 ng/ml using 1640 culture medium containing 10% FBS, followed by 3-fold serial dilutions for a total of 8 gradients. These dilutions of IL2-Fcwt and IL2-Fcmu were added to the culture plate containing CTLL2 cells. After 72 hours of incubation in a $CO_2$ cell culture incubator, CCK8 was used to measure the relative cell number in each well, and the EC50 values were calculated to determine the activity of the samples.

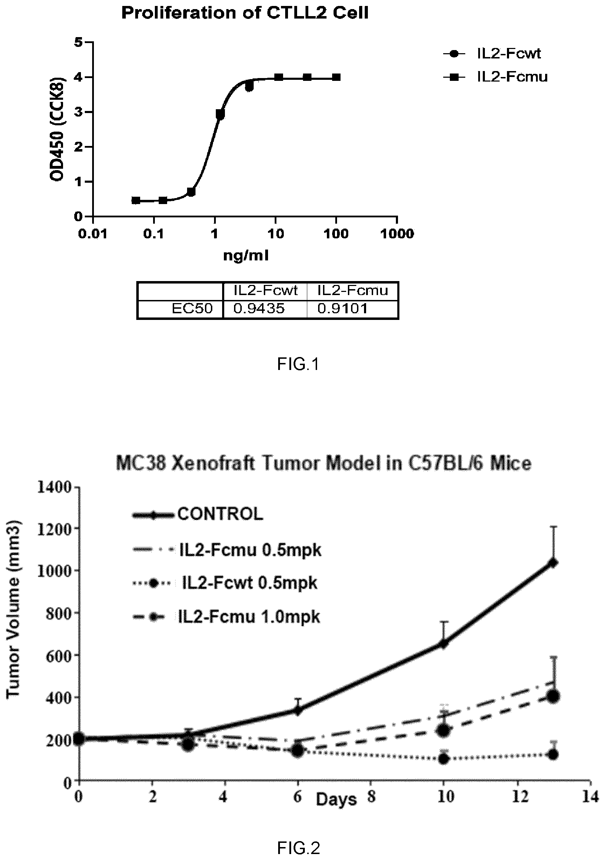

The results, as shown in FIG. 1, demonstrated that both IL2-Fcwt and IL2-Fcmu can stimulate the proliferation of CTLL2 cells, with EC50 values of 0.94 ng/ml and 0.91 ng/ml, respectively, indicating that IL2-Fcmu has almost the same biological activity as IL2-Fcwt.

## 1.3 *In Vivo* Toxicity of IL 2-Fcmu Is Significantly Reduced

From day 0 to day 2, intraperitoneal injection was performed on C57BL/6 mice (purchased from Charles River Company) twice, with a volume of 0.2 ml per injection. IL2-Fcwt was administered at doses of 0.5 mg/kg, 1 mg/kg, and 2 mg/kg, while IL2-Fcmu was administered at doses of 0.5 mg/kg, 1 mg/kg, 2 mg/kg, and 4 mg/kg. On day 7, the mortality of the experimental mice was observed.

TABLE 1 Survival Rate of Experimental Animals in Each Dose Group

| Sample | 0.5mg/kg | 1.0mg/kg | 2.0mg/kg | 4.0mg/kg |
|---|---|---|---|---|
| IL2-Fcwt | 100% | 60% | 0% | - |
| IL2-Fcmu | 100% | 100% | 100% | 70% |

The results are shown in Table 1. On Day 7 of the experiment, in the IL2-Fcwt 2 mg/kg dose group, all experimental animals died (survival rate 0%), in the IL2-Fcwt 1 mg/kg dose group, 40% of the animals died, while in the IL2-Fcwt 0.5 mg/kg dose group, no animals died. In the IL2-Fcmu 4 mg/kg dose group, 30% of the experimental animals died, while no animals died in the other IL2-Fcmu dose groups. These results indicate that the Fc mutation in IL2-Fcmu significantly reduces toxicity for experimental mice.

## 1.4 *In Vivo* Antitumor Activity of IL2-Fcmu

This embodiment evaluates and compares the *in vivo* antitumor activity of IL2-Fcwt and IL2-Fcmu using a mouse colorectal cancer cell MC38 xenograft model. The experimental method was as follows:

The mouse colorectal cancer cell MC38 was collected after being cultured *in vitro,* and the cell suspension concentration was adjusted to $1 \times 10^7$/ml. The fur on the right ribcage of the C57BL/6 mice was shaved. Under sterile conditions, 100$\mu$l of the cell suspension was inoculated subcutaneously into the right ribcage of the C57BL/6 mice. The subcutaneous xenograft tumor in mice was measured for its diameter using a vernier caliper. Once the average tumor volume reached 100-200 mm$^3$, the animals were randomly divided into groups, with 6 mice per group. IL2-Fcwt was administered at a dose of 0.5 mg/kg, and IL2-Fcmu was administered at doses of 0.5 mg/kg and 1.0 mg/kg, all via intraperitoneal injection. An equal volume of PBS was administered to the control group via intraperitoneal injection three times per week, with a volume of 0.2 ml per injection, continuously for 2 weeks. Throughout the entire experiment process, the diameter of the xenograft tumor was measured twice a week, and the body weight of the mice was recorded. The tumor volume (TV) was calculated using the following formula:

$$TV = 1/2 \times a \times b^2$$

wherein a and b represent the length and width, respectively. The relative tumor volume (RTV) was calculated based on the measurements using the formula: RTV = Vt / V0, wherein V0 is the tumor volume measured at the time of grouping (i.e., day 0), and Vt is the tumor volume measured at each subsequent time point. The antitumor activity was evaluated using the relative tumor growth rate T/C (%), which was calculated using the following formula:

$$T/C(\%) = (TRTV/CRTV) \times 100$$

$$\text{Tumor inhibition rate TGI (\%)} = 100 - T/C\ (\%)$$

TRTV: experimental group (which was subjected to treatment) RTV; CRTV: negative control group RTV.

The results, as shown in FIG. 2, indicated that at the 0.5 mg/kg dose, both IL2-Fcwt and IL2-Fcmu exhibited similar and good antitumor activity, with TGI values of 61% and 53%, respectively. Statistical analysis showed no significant difference in antitumor activity between IL2-Fcwt and IL2-Fcmu (p > 0.05). The IL2-Fcmu group at a dose of 1.0 mg/kg achieved a TGI of 88%, which was higher than the IL2-Fcmu group at a dose of 0.5 mg/kg and also significantly higher than the IL2-Fcwt group.

**Embodiment 2 Use of Antibody Mutation Technology in IL12-Fc Fusion Protein**

This embodiment uses one form of IL12-Fc fusion protein, specifically the IL12P40-P35-Fc fusion protein, to illustrate the effect of Fc mutation technology. The Fc-mutated IL12-Fcmu fusion protein significantly reduces *in vivo* toxic side effects while maintaining its *in vivo* and *in vitro* biological activity. This effect is applicable to various forms of IL12-Fc fusion proteins, as described in literature/patents, such as the multiple forms mentioned in Xencor, Inc. patent US 2020/0216509 A1.

**2.1 Preparation of mIL12sc-Fcwt and mIL12sc-Fcmu**

Human IL12 includes two subunits, P35 (amino acid sequence as shown in SEQ ID NO:5) and P40 (amino acid sequence as shown in SEQ ID NO:7). Mouse IL12 also includes two subunits, P35 (amino acid sequence as shown in SEQ ID NO:6) and P40 (amino acid sequence as shown in SEQ ID NO:8). Human IL12 P40 and human IL12 P35 were linked via GSGSSRGGSGSGGSGGGGS to form the human single-chain IL12, referred to as huIL12sc (amino acid sequence as shown in SEQ ID NO:9). Mouse IL12 P40 and mouse IL12 P35 were linked via GSGSSRGGSGSGGSGGGGS to form the mouse single-chain IL12, referred to as mIL12sc (amino acid sequence as shown in SEQ ID NO:10). huIL12sc was linked to human IgG1-Fc (amino acid sequence as shown in SEQ ID NO:14) to form the huIL12sc-Fcwt fusion protein (amino acid sequence as shown in SEQ ID NO:20). huIL12sc was linked to the mutated human IgG1 Fc (amino acid sequence as shown in SEQ ID NO:15) to form the huIL12sc-Fcmu fusion protein (amino acid sequence as shown in SEQ ID NO:22). mIL12sc was linked to human IgG1-Fc (amino acid sequence as shown in SEQ ID NO:14) to form the mIL12sc-Fcwt fusion protein (amino acid sequence as shown in SEQ ID NO:21). mIL12sc was linked to the mutated human IgG1 Fc (amino acid sequence as shown in SEQ ID NO:15) to form the mIL12sc-Fcmu fusion protein (amino acid sequence as shown in SEQ ID NO:23). The genes for huIL12sc-Fcwt, huIL12sc-Fcmu, mIL12sc-Fcwt, and mIL12sc-Fcmu were each constructed into the pcDNA3.4 expression vector, followed by transfected into Expi-293F cells, and purification was performed using Protein G to obtain the fusion proteins huIL12sc-Fcwt, huIL12sc-Fcmu, mIL12sc-Fcwt, and mIL12sc-Fcmu, with a purity of >95%. These fusion proteins were then quantified, aliquoted, and stored at -80°C for later use.

**2.2 *In Vivo* Toxicity of mIL12sc-Femu Is Significantly Reduced**

The method was the same as that in Embodiment 1.3.

TABLE 2 Survival Rate of Experimental Animals in Each Dose Group

| Sample | 0.5mg/kg | 1.0mg/kg | 2.0mg/kg | 4.0mg/kg |
|---|---|---|---|---|
| mIL12sc-Fcwt | 100% | 60% | 0% | - |
| mIL 12sc-Fcmu | 100% | 100% | 100% | 20% |

The results are shown in Table 2. On Day 7 after the first administration, all experimental animals in the mIL12sc-Fcwt 2 mg/kg dose group died (survival rate 0%), in the mIL12sc-Fcwt 1 mg/kg dose group, 40% of the animals died, while in the mIL12sc-Fcwt 0.5 mg/kg dose group, no animals died. In the mIL12sc-Fcmu 4 mg/kg dose group, 80% of experimental animals died, while no animals died in the other dose groups. These results indicate that the Fc mutation in mIL12sc-Fcmu significantly reduces toxicity for experimental mice.

**2.3 mIL12sc-Fcmu Maintains *In Vivo* Antitumor Activity**

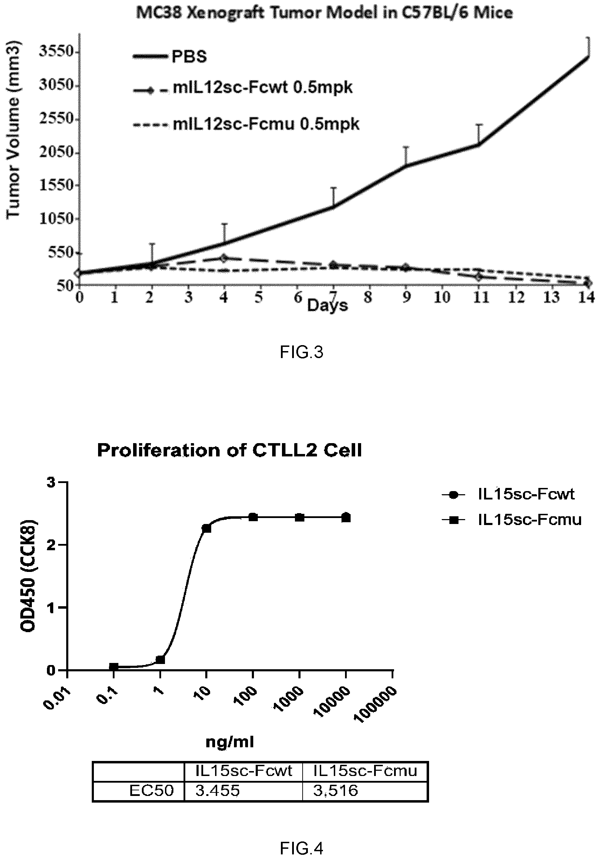

The method was the same as that in Embodiment 1.3. Both mIL12sc-Fcwt and mIL12sc-Fcmu were administered at a dose of 0.5 mg/kg, 3 times per week, for a total of 6 doses.
The results, as shown in FIG. 3, indicated that both mIL12sc-Fcwt and mIL12sc-Fcmu exhibited excellent antitumor activity, with TGI values of 98% and 96%, respectively, and with no significant difference therebetween (p>0.05). This demonstrates that the Fc mutation in mIL12sc-Fcmu does not affect the *in vivo* antitumor activity.

**Embodiment 3 Use of Antibody Mutation Technology in IL15-Fc Fusion Protein**

This embodiment uses one form of IL15-Fc fusion protein, specifically the IL15Rsushi-IL15-Fc fusion protein, to illustrate the effect of Fc mutation technology. The Fc-mutated IL15-Fc fusion protein significantly reduces *in vivo* toxic side effects while maintaining its *in vivo* and *in vitro* biological activity. This effect is applicable to various forms of IL15-Fc fusion proteins, as described in literature/patents, such as the multiple forms mentioned in the Xencor, Inc. patent US 2018/0118805 A1.

**3.1 Preparation of Fusion Proteins hRsushi-hIL15-Fcwt and hRsushi-hIL15-Fcmu**

Human IL15Rsushi (amino acid sequence as shown in SEQ ID NO:12) was linked to human IL15 (amino acid sequence as shown in SEQ ID NO:11) via GGGGSGGGGSGGGGSGGGGSGGGGSGGGGS to form the single-chain IL15, referred to as huIL15sc (amino acid sequence as shown in SEQ ID NO:13). huIL15sc was linked to human IgG1 Fc (amino acid sequence as shown in SEQ ID NO:14) to form the fusion protein hIL15sc-Fcwt (amino acid sequence as shown in SEQ ID NO:24). huIL15sc was linked to the mutated human IgG1 Fc (amino acid sequence as shown in SEQ ID NO:15) to form the fusion protein hIL15sc-Fcmu (amino acid sequence as shown in SEQ ID NO:25). The genes for hIL15sc-Femu and hIL15sc-Fewt were each constructed into the pcDNA3.4 expression vector, followed by transfected into Expi-293F cells, and purification was performed using Protein G to obtain the fusion proteins hIL15sc-Fcmu and hIL15sc-Fewt, with a purity of >95%. The fusion proteins were then quantified, aliquoted, and stored at - 80°C for later use.

**3.2 *In Vitro* Biological Activity of hIL15sc-Fcmu**

The biological activity of hIL15sc-Fcmu and hIL15sc-Fcwt was measured using a CTLL2 cell proliferation assay. The method was the same as that in Embodiment 1.2. The dilution gradient for both IL15sc-Fcwt and IL15sc-Fcmu started from 10000 ng/ml, with a 10-fold serial dilution.
The results, as shown in FIG. 4, demonstrated that both IL15sc-Fcwt and IL15sc-Fcmu can stimulate the proliferation of CTLL2 cells, with EC50 values of 3.455 ng/ml and 3.516 ng/ml, respectively, indicating that IL15sc-Fcmu has almost the same biological activity as IL15sc-Fcwt, as shown in Table 3.1 below.

TABLE 3.1

| ng/ml | IL15sc-Fcwt | IL15sc-Fcmu |
|---|---|---|
| 10000 | 2.459 | 2.428 |
| 1000 | 2.446 | 2.438 |
| 100 | 2.453 | 2.44 |
| 10 | 2.268 | 2.256 |

(continued)

| ng/ml | IL15sc-Fcwt | IL15sc-Fcmu |
|---|---|---|
| 1 | 0.174 | 0.169 |
| 0.1 | 0.049 | 0.058 |

**3.3 *In Vivo* Toxic Side Effect of hIL15sc-Fcmu Is Significantly Reduced Compared to hIL15sc-Fcwt**

The method was the same as that in Embodiment 1.3.

TABLE 3 Survival Rate of Experimental Animals in Each Dose Group

| Sample | 0.5mg/kg | 1.0mg/kg | 2.0mg/kg | 4.0mg/kg |
|---|---|---|---|---|
| hIL15sc-Fcwt | 100% | 80% | 0% | - |
| hIL15sc-Fcmu | 100% | 100% | 100% | 40% |

The results are shown in Table 3. On Day 7 of the experiment, in the hIL15sc-Fcwt 2 mg/kg dose group, all experimental animals died (survival rate 0%), in the hIL15sc-Fcwt 1 mg/kg dose group, 20% of the animals died, while in the hIL15sc-Fcwt 0.5 mg/kg dose group, no animals died. In the hIL15sc-Fcmu 4 mg/kg dose group, 60% of the experimental animals died, while no animals died in the other dose groups. These results indicate that the Fc mutation in hIL15sc-Fcmu significantly reduces toxicity for experimental mice.

**3.4 *In Vivo* Antitumor Activity of hIL15sc-Fcmu**

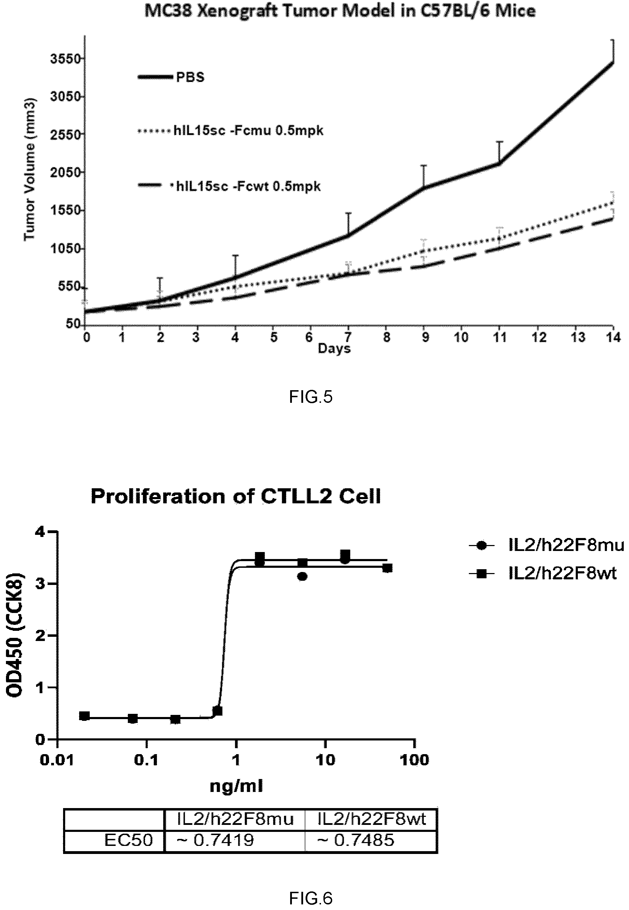

The method was the same as that in Embodiment 1.3. Both hIL15sc-Fcwt and hIL15sc-Fcmu were administered at a dose of 0.5 mg/kg, 3 times per week, for a total of 6 doses.
The results, as shown in FIG. 5, indicated that both hIL15sc-Fcwt and hIL15sc-Fcmu exhibited good antitumor activity, with TGI values of 53% and 58%, respectively. Statistical analysis showed no significant difference in antitumor activity between hIL15sc-Fcwt and hIL15sc-Femu ($p > 0.05$), demonstrating that the Fc mutation hIL15sc-Fcmu maintained its good *in vivo* antitumor activity.

**Embodiment 4 Use of Fc Mutation Technology in IL2/Monoclonal Antibody Complex**

This embodiment uses human IL2 antibody hu22F8 prepared by ourselves to illustrate that the complex (IL2/hu22F8mu), which is formed by the Fc-mutated antibody hu22F8mu and human IL2 proleukin (amino acid sequence as shown in SEQ ID NO: 2), significantly reduces the *in vivo* toxicity, while retaining the *in vitro* and *in vivo* biological activity of the complex (IL2/hu22F8wt), which is formed by the wild-type antibody hu22F8wt and human IL2. This Fc mutation technology, when applied to other types of IL2/monoclonal antibody complexes, can achieve the same effect, such as the IL2 monoclonal antibody NARA1 described in the literature.

**4.1 Preparation of Humanized IL2 Monoclonal Antibody hu22F8mu**

The amino acid sequence of the heavy chain of the humanized IL2 monoclonal antibody hu22F8wt is as shown in SEQ ID NO:27. The amino acid sequence of the heavy chain of the Fc-mutated humanized IL2 monoclonal antibody hu22F8mu is as shown in SEQ ID NO:26. The amino acid sequence of the light chain of the humanized IL2 monoclonal antibody hu22F8wt and hu22F8mu is as shown in SEQ ID NO:28. The amino acid sequence of human IL2 is as shown in SEQ ID NO:2.
The genes for the heavy chain, mutated heavy chain, and light chain were each constructed into the pcDNA3.4 expression vector, followed by co-transfected into Expi-293F cells, and purification was performed using Protein G to obtain the antibodies hu22F8wt and hu22F8mu. The molecular weight of each expressed antibody was determined to be around 150 kDa by SDS-PAGE electrophoresis and SEC-HPLC. The antibody purity was >95%, and the antibodies were quantified, aliquoted, and stored at - 80°C for later use.

**4.2 *In Vitro* Biological Activity of Human IL2/hu22F8mu Complex**

The biological activity of IL2/hu22F8mu complex was measured using a CTLL2 cell proliferation assay. The method was similar to Example 1.2. IL2 was mixed with hu22F8mu or hu22F8wt at a mass ratio of 1:7 and incubated at room temperature for 30 minutes to form the IL2/hu22F8mu complex or IL2/hu22F8wt complex. The IL2/hu22F8mu complex and IL2/hu22F8wt complex were then diluted to 50 ng/ml based on IL2 concentration, followed by 3-fold serial dilutions for a total of 8 gradients. Complexes with each gradient were added to the culture plates containing CTLL2 cells.

The results, as shown in FIG. 6, demonstrated that both IL2/hu22F8mu and IL2/hu22F8wt can stimulate the proliferation of CTLL2 cells, with EC50 values of 0.7419 ng/ml and 0.7485 ng/ml, respectively, indicating that IL2/hu22F8-mu has almost the same biological activity as IL2/hu22F8wt.

### 4.3 *In Vivo* Toxicity of Human IL2/hu22F8mu Complex Is Significantly Reduced

The preparation method of the IL2/hu22F8wt complex and IL2/hu22F8mu complex was as follows: IL2 was mixed with hu22F8wt or hu22F8mu at a mass ratio of 1:7, incubated at room temperature for 30 minutes, and then performed administration. The remaining procedures were the same as those in Example 1.3, with the dosing based on IL2.

TABLE 4 Survival Rate of Experimental Animals in Each Antibody Dose Group

| Sample | 0.5mg/kg | 1.0mg/kg | 2.0mg/kg | 4.0mg/kg |
|---|---|---|---|---|
| IL2/hu22F8wt | 100% | 40% | 0% | - |
| IL2/hu22F8mu | 100% | 100% | 100% | 1000% |

The results are shown in Table 4. On Day 7 of the experiment, all experimental animals in the IL2/hu22F8wt 2 mg/kg dose group died (survival rate 0%), in the IL2/hu22F8wt 1 mg/kg dose group, 60% of the animals died, while in the IL2/hu22F8wt 0.5 mg/kg dose group, no animals died. In the IL2/hu22F8mu group at the highest dose of 4 mg/kg, no experimental animals died, and no animals died in the other dose groups either. These results indicate that the Fc mutation in IL2/hu22F8mu significantly reduces toxicity for experimental mice.

### 4.4 *In Vivo* Antitumor Activity of Human IL2/hu22F8mu Complex

This embodiment evaluates and compares the *in vivo* antitumor activity of the IL2/hu22F8mu complex and the IL2/hu22F8wt complex using a mouse colorectal cancer cell MC38 xenograft model. The experimental method was similar to that in Example 1.4.

The IL2/hu22F8mu complex was prepared as described above, with doses of 1 mg/kg and 0.5 mg/kg based on IL2.

The IL2/hu22F8wt complex was prepared as described above, with a dose of 0.5mg/kg based on IL2.

The IL2/hu22F8mu complex and the IL2/hu22F8wt complex were administered by intraperitoneal injection three times per week.

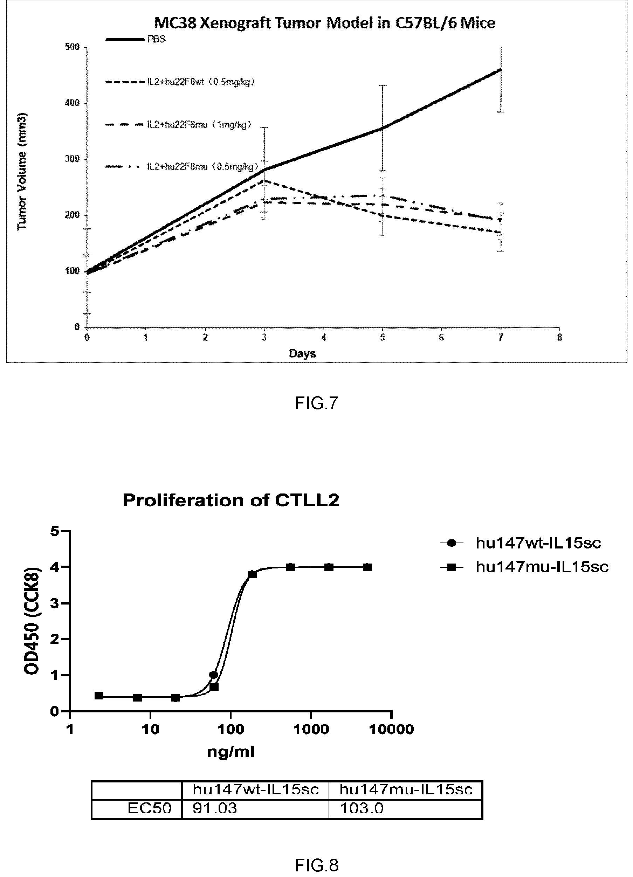

The results, as shown in FIG. 7, indicated that the IL2/hu22F8mu complex and the IL2/hu22F8wt complex both exhibited excellent antitumor activity. After three injections (1 week), the TGI of the IL2/hu22F8mu group at doses of 1 mg/kg and 0.5 mg/kg reached 58% and 59%, respectively, the TGI of the IL2/hu22F8wt group at a dose of 0.5 mg/kg reached 63%. Statistical analysis showed no significant difference in antitumor activity between these groups ($p > 0.05$).

This result is consistent with the results of Embodiment 4.3, indicating that the toxicity of the IL2/hu22F8mu complex is significantly lower than that of the IL2/hu22F8wt complex.

### Embodiment 5 Use of Antibody Mutation Technology in CD276 Monoclonal Antibody-IL15 Bifunctional Molecule

Taking humanized CD276 monoclonal antibody hu147 prepared by ourselves as an example, it is demonstrated that the mutation of Fc in the monoclonal antibody of the bispecific molecule formed by CD276 monoclonal antibody and IL15 can reduce the *in vivo* toxicity of this bispecific molecule, thereby leading to improved safety for wider applications and better therapeutic efficacy by increasing the dose. This effect is also applicable to other CD276 monoclonal antibodies.

### 5.1 Preparation of Bifunctional Molecules hu147wt-hIL15sc and hu147mu-hIL15sc

The heavy chain of the humanized CD276 monoclonal antibody hu147wt (amino acid sequence as shown in SEQ ID NO:29) was linked to huIL15sc (constructed as in Embodiment 3, amino acid sequence shown in SEQ ID NO:13) via GGGGSGGGGSGGGGS to form the heavy chain of hu147wt-IL15sc (amino acid sequence as shown in SEQ ID NO:30). The heavy chain of the Fc-mutated humanized CD276 monoclonal antibody hu147mu (amino acid sequence as shown in SEQ ID NO:31) was linked to huIL15sc via GGGGSGGGGSGGGGS to form the heavy chain of hu147mu-hIL15sc (amino acid sequence as shown in SEQ ID NO:32). The amino acid sequence of the light chain of hu147wt/hu147-mu is as shown in SEQ ID NO:33. The genes for the heavy chain, mutated heavy chain, and light chain were each constructed into the pcDNA3.4 expression vector, followed by co-transfected into Expi-293F cells, and purification was performed using Protein G to obtain the bifunctional molecules hu147wt-hIL15sc and hu147mu-hIL15sc, with a purity of >95%. The bifunctional molecules were then quantified, aliquoted, and stored at -80°C for later use.

### 5.2 *In Vitro* Biological Activity of Bifunctional Molecule hu147mu-hIL15sc

The experimental method was the same as that in Embodiment 1.2, using the CTLL2 cell proliferation assay to measure the biological activity of hu147mu-IL15sc and hu147wt-IL15sc.

The results, as shown in FIG. 8, demonstrated that both hu147mu-IL15sc and hu147wt-IL15sc can stimulate the proliferation of CTLL2 cells, with EC50 values of 103.0 ng/ml and 91.0 ng/ml, respectively, indicating that hu147mu-IL15sc has almost the same biological activity as hu147wt-IL15sc.

### 5.3. *In Vivo* Toxicity of Bifunctional Molecule hu147mu-hIL15sc Is Significantly Reduced

The method was the same as that in Embodiment 1.3.

TABLE 5 Survival Rate of Experimental Animals in Each Dose Group

| Sample | 0.5mg/kg | 1.0mg/kg | 2.0mg/kg | 4.0mg/kg |
|---|---|---|---|---|
| hu147wt-hIL15sc | 100% | 40% | 0% | 0% |
| hu147mu-hIL15sc | 100% | 100% | 100% | 30% |

The results are shown in Table 5. On Day 7 of the experiment, in the hu147wt-hIL15sc 2 mg/kg dose group and 4 mg/kg dose group, all experimental animals died (survival rate 0%), in the hu147wt-hIL15sc 1 mg/kg dose group, 60% of the animals died, while in the hu147wt-hIL15sc 0.5 mg/kg dose group, no animals died. In the hu147mu-hIL15sc group at the highest dose of 4 mg/kg, 70% of the experimental animals died, while no animals died in the other dose groups. These results indicate that the Fc mutation in hu147mu-hIL15sc significantly reduces toxicity for experimental mice.

### 5.4 *In Vivo* Antitumor Activity of Bifunctional Molecule hu147mu-hIL15sc

The method was the same as that in Embodiment 1.3. Both hu147mu-hIL15sc and hu147wt-hIL15sc were administered at a dose of 0.5 mg/kg, 3 times per week, for a total of 6 doses.

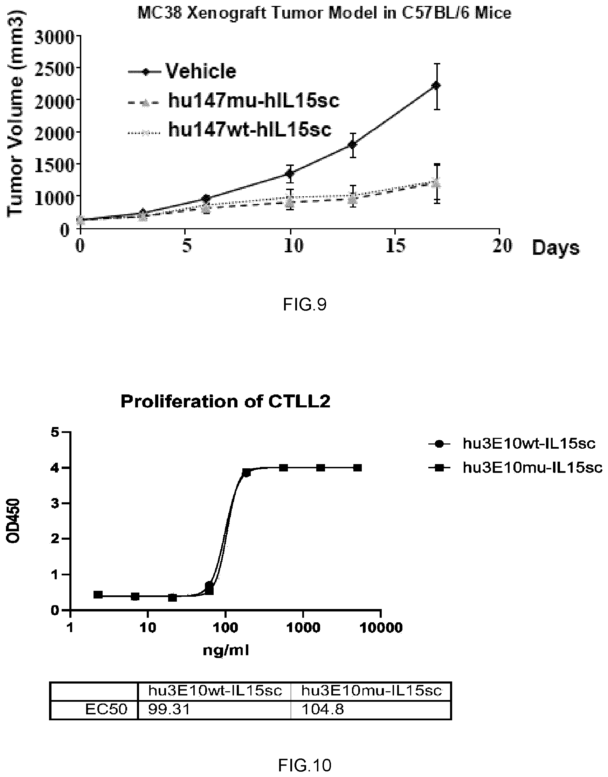

The results, as shown in FIG. 9, indicated that both hu147mu-hIL15sc and hu147wt-hIL15sc exhibited excellent antitumor activity, with TGI values of 69% and 67%, respectively, and with no significant difference therebetween (p>0.05). This demonstrates that the Fc mutation in hu147mu-hIL15sc does not affect *in vivo* antitumor activity.

### Embodiment 6 Use of Antibody Mutation Technology in CD73 Monoclonal Antibody-IL15 Bifunctional Molecule

Taking humanized CD73 monoclonal antibody hu3E10 prepared by ourselves as an example, it is demonstrated that the mutation of Fc in the monoclonal antibody of the bispecific molecule formed by CD73 monoclonal antibody and IL15 can reduce the *in vivo* toxicity of this bispecific molecule, thereby leading to improved safety for wider applications and better therapeutic efficacy by increasing the dose. This effect is also applicable to other CD73 monoclonal antibodies.

### 6.1 Preparation of Bifunctional Molecules hu3E10wt-IL15sc and hu3E10mu-IL15sc

The heavy chain of the humanized CD73 monoclonal antibody hu3E10wt (amino acid sequence as shown in SEQ ID NO: 34) was linked to huIL15sc (constructed as in Embodiment 3, amino acid sequence as shown in SEQ ID NO: 13) via GGGGSGGGGSGGGGS to form the heavy chain of hu3E10wt-IL15sc (amino acid sequence as shown in SEQ ID NO: 35). The heavy chain of the Fc-mutated humanized CD73 monoclonal antibody hu3E10mu (amino acid sequence as shown in SEQ ID NO: 36) was linked to huIL15sc via GGGGSGGGGSGGGGS to form the heavy chain of hu3E10mu-IL15sc (amino acid sequence as shown in SEQ ID NO: 37). The amino acid sequence of the light chain of hu3E10wt/hu3E10mu is shown in SEQ ID NO: 38. The genes for the heavy chain, mutated heavy chain, and light chain were each constructed into the pcDNA3.4 expression vector, followed by co-transfected into Expi-293F cells, and purification was performed using Protein G to obtain the bifunctional molecules hu3E10wt-hIL15sc and hu3E10mu-hIL15sc, with a purity of >95%. The bifunctional molecules were quantified, aliquoted, and stored at -80°C for later use.

### 6.2 *In Vitro* Biological Activity of Bifunctional Molecule hu3E10mu-hIL15sc

The experimental method was the same as that in Embodiment 1.2, using the CTLL2 cell proliferation assay to measure the biological activity of hu3E10mu-IL15sc and hu3E10wt-IL15sc.

The results, as shown in FIG. 10, demonstrated that both hu3E10mu-IL15sc and hu3E10wt-IL15sc can stimulate the proliferation of CTLL2 cells, with EC50 values of 104.8 ng/ml and 99.3 ng/ml, respectively, indicating that hu3E10mu-IL15sc has almost the same biological activity as hu3E10wt-IL15sc.

### 6.3 *In Vivo* Toxicity of Bifunctional Molecule hu3E10mu-hIL15sc Is Significantly Reduced

The method was the same as that in Embodiment 1.3.

TABLE 6 Survival Rate of Experimental Animals in Each Dose Group

| Sample | 0.5mg/kg | 1.0mg/kg | 2.0mg/kg | 4.0mg/kg |
|---|---|---|---|---|
| hu3E10wt-hIL15sc | 100% | 50% | 0% | 0% |
| hu3E10mu-hIL15sc | 100% | 100% | 100% | 10% |

The results are shown in Table 6. On Day 7 of the experiment, in the hu3E10wt-hIL15sc 4 mg/kg dose group and 2 mg/kg dose group, all experimental animals died (survival rate 0%), in the hu3E10wt-hIL15sc 1 mg/kg dose group, 50% of the animals died, while in the hu3E10wt-hIL15sc 0.5 mg/kg dose group, no animals died. In the hu3E10mu-hIL15sc group at the highest dose of 4 mg/kg, 90% of the experimental animals died, while no animals died in the other dose groups. These results indicate that the Fc mutation in hu3E10mu-hIL15sc significantly reduces toxicity for experimental mice.

### 6.4 *In Vivo* Antitumor Activity of Bifunctional Molecule hu3E10mu-hIL15sc

The method was the same as that in Embodiment 1.3. Both hu3E10mu-hIL15sc and hu3E10wt-hIL15sc were administered at a dose of 0.5 mg/kg, 3 times per week, for a total of 6 doses.

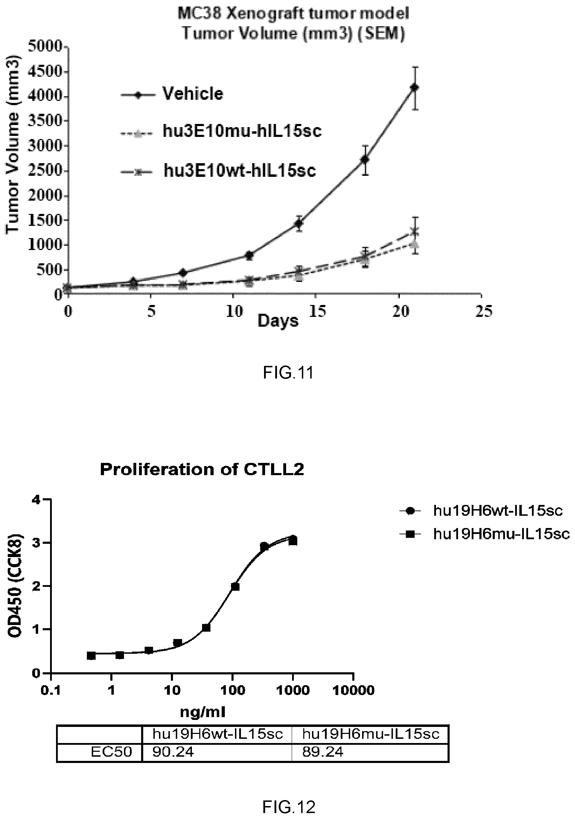

The results, as shown in FIG. 11, indicated that both hu3E10mu-hIL15sc and hu3E10wt-hIL15sc exhibited excellent antitumor activity, with TGI values of 70% and 75%, respectively, with no significant difference therebetween (p>0.05). This demonstrates that the Fc mutation in hu3E10mu-hIL15sc does not affect *in vivo* antitumor activity.

### Embodiment 7 Use of Antibody Mutation Technology in HER2 Monoclonal Antibody-IL15 Bifunctional Molecule

Taking HER2 monoclonal antibody hu19H6 prepared by ourselves as an example, it is demonstrated that the mutation of Fc in the monoclonal antibody of the bispecific molecule formed by the HER2 monoclonal antibody and IL 15 can reduce the *in vivo* toxicity of this bispecific molecule, thereby leading to improved safety for wider applications and better therapeutic efficacy by increasing the dose. This effect is also applicable to other HER2 monoclonal antibodies.

### 7.1 Preparation of Bifunctional Molecule hu19H6-IL15 and hu19H6mu-IL5

The heavy chain of the humanized HER2 monoclonal antibody hu19H6wt (amino acid sequence as shown in SEQ ID NO: 39) was linked to huIL15sc (constructed as in Embodiment 3, amino acid sequence as shown in SEQ ID NO: 13) via GGGGSGGGGSGGGGS to form the heavy chain of hu19H6wt-IL15sc (amino acid sequence as shown in SEQ ID NO: 40). The heavy chain of the Fc-mutated humanized HER2 monoclonal antibody hu19H6mu (amino acid sequence as shown in SEQ ID NO: 41) was also linked to huIL15sc via GGGGSGGGGSGGGGS to form the heavy chain of hu19H6mu-IL15sc (amino acid sequence as shown in SEQ ID NO: 42). The amino acid sequence of the light chain of hu19H6wt/hu19H6mu is as shown in SEQ ID NO: 43. The genes for the heavy chain, mutated heavy chain, and light chain were each constructed into the pcDNA3.4 expression vector, followed by co-transfected into Expi-293F cells, and purification was performed using Protein G to obtain the bifunctional molecules hu19H6wt-hIL15sc and hu19H6mu-hIL15sc, with a purity of >95%. The bifunctional molecules were quantified, aliquoted, and stored at -80°C for later use.

**7.2 *In Vitro* Biological Activity of Bifunctional Molecule hu19H6mu-IL15sc**

The experimental method was the same as that in Embodiment 1.2, using the CTLL2 cell proliferation assay to measure the biological activity of hu19H6mu-IL15sc and hu19H6wt-IL15sc.

The results, as shown in FIG. 12, demonstrated that both hu19H6mu-IL15sc and hu19H6wt-IL15sc can stimulate the proliferation of CTLL2 cells, with EC50 values of 89.24 ng/ml and 90.24 ng/ml, respectively, indicating that hu19H6mu-IL15sc has almost the same biological activity as hu19H6wt-IL15sc.

**7.3 *In Vivo* Toxicity of Bifunctional Molecule hu19H6mu-IL5 Is Significantly Reduced**

The method was the same as that in Embodiment 1.3.

TABLE 7 Survival Rate of Experimental Animals in Each Dose Group

| Sample | 0.5mg/kg | 1.0mg/kg | 2.0mg/kg | 4.0mg/kg |
|---|---|---|---|---|
| hu19H6wt-IL15sc | 100% | 80% | 10% | 0% |
| hu19H6mu-IL15sc | 100% | 100% | 100% | 20% |

The results are shown in Table 7. On Day 7 of the experiment, in the hu19H6wt-hIL15sc 4 mg/kg dose group, all experimental animals died (survival rate 0%), in the hu19H6wt-hIL15sc 2 mg/kg dose group, 90% of the animals died (survival rate 10%), and in the hu19H6wt-hIL15sc 1 mg/kg dose group, 20% of the animals died, while in the hu19H6wt-hIL15sc 0.5 mg/kg dose group, no animals died. In the hu19H6mu-hIL15sc group at the highest dose of 4 mg/kg, 80% of the experimental animals died, while no animals died in the other dose groups. These results indicate that the Fc mutation in hu19H6mu-hIL15sc significantly reduces toxicity for experimental mice.

**7.4 *In Vivo* Antitumor Activity of Bifunctional Molecule hu19H6mu-hIL15sc**

The method was the same as that in Embodiment 1.3. Both hu19H6mu-hIL15sc and hu19H6wt-hIL15sc were administered at a dose of 0.5 mg/kg, 3 times per week, for a total of 6 doses.

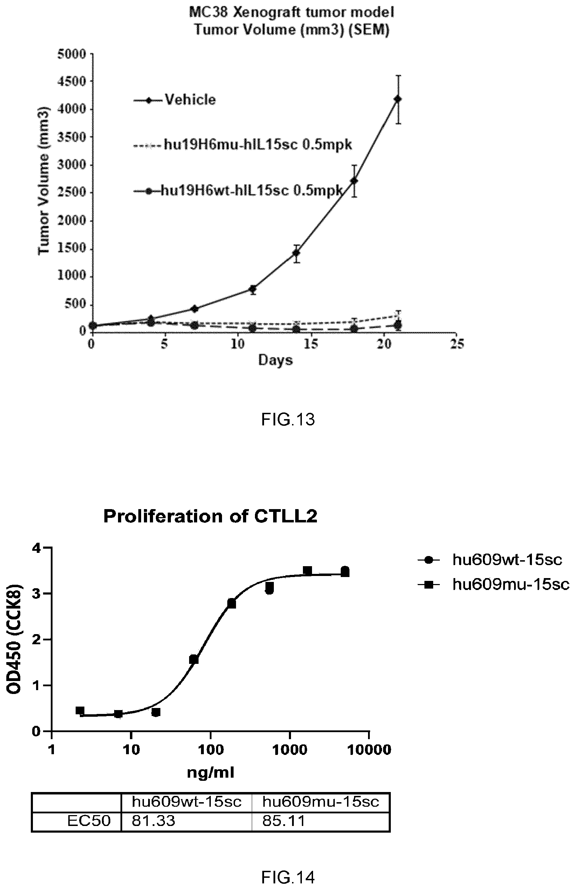

The results, as shown in FIG. 13, indicated that both hu19H6mu-hIL15sc and hu19H6wt-hIL15sc exhibited excellent antitumor activity, with TGI values of 93% and 95%, respectively, with no significant difference therebetween ($p > 0.05$). This demonstrates that the Fc mutation in hu19H6mu-hIL15sc does not affect *in vivo* antitumor activity.

**Embodiment 8 Use of Antibody Mutation Technology in PD1 Monoclonal Antbody-IL15 Bifunctional Molecule**

Taking PD1 monoclonal antibody hu609 prepared by ourselves as an example, it is demonstrated that the mutation of Fc in the monoclonal antibody of the bispecific molecule formed by PD1 monoclonal antibody and IL15 can reduce the *in vivo* toxicity of this bispecific molecule, thereby achieving improved safety for wider applications and better therapeutic efficacy by increasing the dose.

**8.1 Preparation of Bifunctional Molecules hu609-IL15 and hu609mu-IL15**

The heavy chain of the humanized PD1 monoclonal antibody hu609wt (amino acid sequence as shown in SEQ ID NO: 44) was linked to huIL15sc (constructed as in Embodiment 3, amino acid sequence as shown in SEQ ID NO: 13) via GGGGSGGGGSGGGGS to form the heavy chain of hu609wt-IL15sc (amino acid sequence as shown in SEQ ID NO: 45). The heavy chain of the Fc-mutated humanized PD1 monoclonal antibody hu609mu (amino acid sequence as shown in SEQ ID NO: 46) was also linked to huIL15sc via GGGGSGGGGSGGGGS to form the heavy chain of hu609mu-IL15sc (amino acid sequence as shown in SEQ ID NO: 47). The amino acid sequence of the light chain of hu609wt/hu609mu is as shown in SEQ ID NO: 48. The genes for the heavy chain, mutated heavy chain, and light chain were each constructed into the pcDNA3.4 expression vector, followed by co-transfected into Expi-293F cells, and purification was performed using Protein G to obtain the bifunctional molecules hu609wt-hIL15sc and hu609mu-hIL15sc, with a purity of >95%. The bifunctional molecules were quantified, aliquoted, and stored at -80°C for later use.

**8.2 *In Vitro* Biological Activity of Bifunctional Molecule hu609mu-IL15sc**

The experimental method was the same as that in Embodiment 1.2, using the CTLL2 cell proliferation assay to measure the biological activity of hu609mu-IL15sc and hu609wt-IL15sc.

The results, as shown in FIG. 14, demonstrated that both hu609mu-IL15sc and hu609wt-IL5sc can stimulate the proliferation of CTLL2 cells, with EC50 values of 85.11 ng/ml and 81.33 ng/ml, respectively, indicating that hu609mu-IL15sc has almost the same biological activity as hu609wt-IL15sc.

### 8.3 *In Vivo* Toxicity of Bifunctional Molecule hu609mu-IL15sc is Significantly Reduced

The method was the same as that in Embodiment 1.3.

TABLE 8 Survival Rate of Experimental Animals in Each Dose Group

| Sample | 0.5mg/kg | 1.0mg/kg | 2.0mg/kg | 4.0mg/kg |
|---|---|---|---|---|
| Hu609wt-hIL15sc | 100% | 60% | 0% | 0% |
| Hu609mu-IL15sc | 100% | 100% | 100% | 10% |

The results are shown in Table 8. On Day 7 of the experiment, in the hu609wt-hIL15sc 4 mg/kg dose group and 2 mg/kg dose group, all experimental animals died (survival rate 0%), in the hu609wt-hIL15sc 1 mg/kg dose group, 40% of the animals died, while in the hu609wt-hIL15sc 0.5 mg/kg dose group, no animals died.
**In** the hu609mu-IL15sc group at the highest dose of 4 mg/kg, 90% of the experimental animals died, while no animals died in the other dose groups. These results indicate that the Fc mutation in hu609mu-IL15sc significantly reduces toxicity for experimental mice.

### 8.4 *In Vivo* Antitumor Activity of Bifunctional Molecule hu609mu-IL15sc

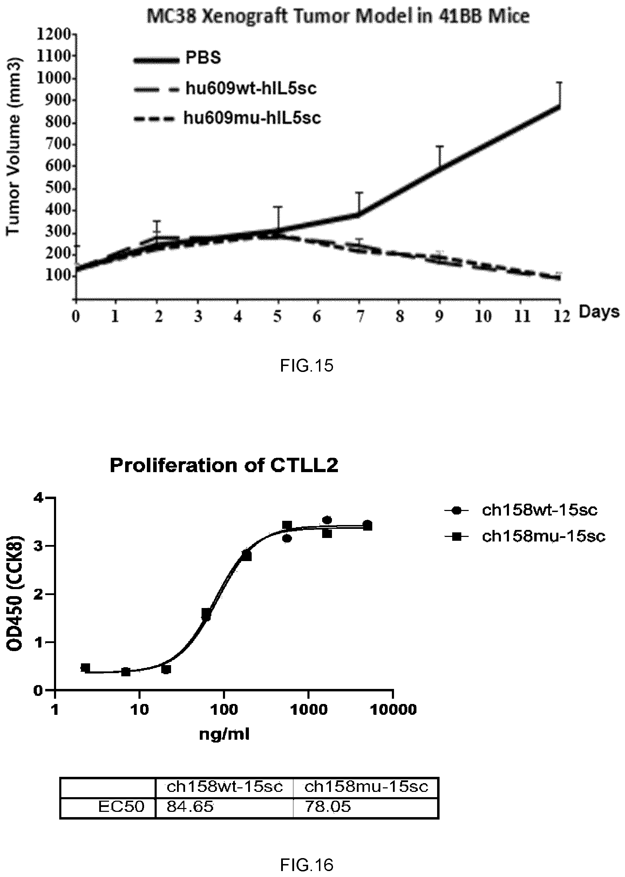

The method was the same as Embodiment 1.3. Both hu609mu-IL15sc and hu609wt-IL15sc were administered at a dose of 0.5 mg/kg, 3 times per week, for a total of 6 doses.
The results, as shown in FIG. 15, indicated that both hu609mu-IL15sc and hu609-IL15sc exhibited excellent antitumor activity, with TGI values of 89.6% and 89.3%, respectively, with no significant difference therebetween (p>0.05). This demonstrates that the Fc mutation in hu609mu-IL15 does not affect *in vivo* antitumor activity

### Embodiment 9 Use of Antibody Mutation Technology in HER3 Monoclonal Antibody-IL15sc Bifunctional Molecule

Taking HER3 chimeric monoclonal antibody ch158 prepared by ourselves as an example, it is demonstrated that the mutation of Fc in the monoclonal antibody of the bispecific molecule formed by HER3 monoclonal antibody and IL15 can reduce the *in vivo* toxicity of this bispecific molecule, thereby achieving improved safety for wider applications and better therapeutic efficacy by increasing the dose. This effect is also applicable to other HER3 monoclonal antibodies.

### 9.1 Preparation of Bifunctional Molecules ch158wt-IL15sc and ch158mu-IL15sc

The heavy chain of the HER3 monoclonal antibody ch158wt (amino acid sequence as shown in SEQ ID NO: 49) was linked to huIL15sc (constructed as in Example 3, amino acid sequence as shown in SEQ ID NO: 13) via GGGGSGGGGSGGGGS to form the heavy chain of ch158wt-IL15sc (amino acid sequence as shown in SEQ ID NO: 50). The heavy chain of the Fc-mutated HER3 monoclonal antibody ch158mu (amino acid sequence as shown in SEQ ID NO: 51) was linked to huIL15sc via GGGGSGGGGSGGGGS to form the heavy chain of ch158mu-IL15sc (amino acid sequence as shown in SEQ ID NO: 52). The amino acid sequence of the light chain of ch158wt/ch158mu is as shown in SEQ ID NO: 53. The genes for the heavy chain, mutated heavy chain, and light chain were each constructed into the pcDNA3.4 expression vector, followed by co-transfected into Expi-293F cells, and purification was performed using Protein G to obtain the bifunctional molecules ch158wt-hIL15sc and ch158mu-hIL15sc, with a purity of >95%. The bifunctional molecules were quantified, aliquoted, and stored at -80°C for later use.

### 9.2 *In Vitro* Biological Activity of Bifunctional Molecule ch158mu-IL15sc

The experimental method was the same as that in Embodiment 1.2, using the CTLL2 cell proliferation assay to measure the biological activity of ch158mu-IL15sc and ch158wt-IL15sc.
The results, as shown in FIG. 16, demonstrated that both ch158mu-IL15sc and ch158wt-IL5sc can stimulate the proliferation of CTLL2 cells, with EC50 values of 78.05 ng/ml and 84.65 ng/ml, respectively, indicating that ch158mu-IL15sc has almost the same biological activity as ch158wt-IL15sc.

### 9.3 *In Vivo* Toxicity of Bifunctional Molecule ch158mu-IL15sc is Significantly Reduced

The method was the same as that in Embodiment 1.3.

TABLE 9 Survival Rate of Experimental Animals in Each Dose Group

| Sample | 0.5mg/kg | 1.0mg/kg | 2.0mg/kg | 4.0mg/kg |
|---|---|---|---|---|
| ch158wt-IL15 | 100% | 70% | 20% | 10% |
| ch158mu-IL15 | 100% | 100% | 100% | 30% |

The results are shown in Table 9. On Day 7 of the experiment, in the ch158wt-hIL15sc 4 mg/kg dose group, 90% of the animals died (survival rate 10%), in the ch158wt-hIL15sc 2 mg/kg dose group, 80% of the animals still died, and in the ch158wt-hIL15sc 1 mg/kg dose group, 30% of the animals died, while in the ch158wt-hIL15sc 0.5 mg/kg dose group, no animals died.

In the ch158mu-hIL15sc group at the highest dose of 4 mg/kg, 70% of the experimental animals died, while no animals died in the other dose groups. These results indicate that the Fc mutation in ch158mu-hIL15sc significantly reduces toxicity for experimental mice.

### 9.4 *In Vivo* Antitumor Activity of Bifunctional Molecule ch158mu-hIL15sc

The method was the same as that in Embodiment 1.3. Both ch158mu-hIL15sc and ch158wt-hIL15sc were administered at a dose of 0.5 mg/kg, 3 times per week, for a total of 6 doses.

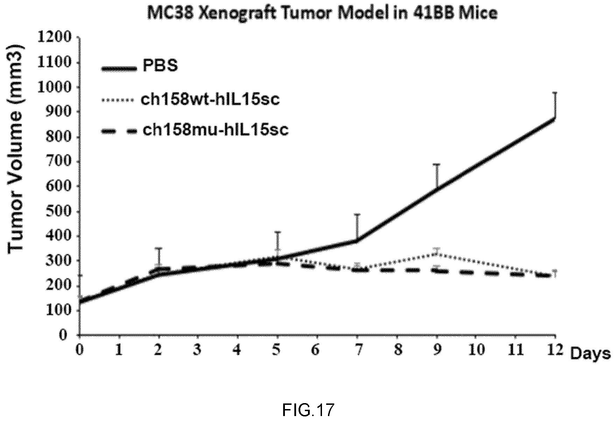

The results, as shown in FIG. 17, indicated that both ch158mu-lhL15sc and ch158wt-hIL15sc exhibited excellent antitumor activity, with TGI values of 72.9% and 72.7%, respectively, with no significant difference therebetween (p>0.05). This demonstrates that the Fc mutation in ch158mu-hIL15sc does not affect *in vivo* antitumor activity.

### Embodiment 10 Other Tumor Targeting Fc-Mutated Antibody-IL15sc Bifunctional Molecules

The Fc-mutant antibodies in the bifunctional molecules, including those targeting antigens related to tumor treatment, such as IL2, PD1, HER2, CD73, CD276, and HER3, significantly reduce *in vivo* toxic side effects while maintaining antitumor activity.

The application of the Fc mutation in the present disclosure is not limited to the antibodies targeting the aforementioned antigens. The Fc mutation can also be applied to antibodies targeting other antigens, such as EGFR, PDL1, CD19, CD20, CD22, CD33, VEGFR, TIGIT, TIM3, LAG3, CXCR3, CXCR5, CCR3, CCR4, CCR9, CD40, CD40L, CD24, etc. The bifunctional molecules formed by these antibodies and IL15 can also achieve the goal of reducing toxic side effects through the Fc mutation.

Taking Rituximab as an example, the amino acid sequence of the heavy chain of the bifunctional molecule formed by the Fc-mutated CD20 antibody and IL15 is as shown in SEQ ID NO: 54; the amino acid sequence of the light chain is as shown in SEQ ID NO: 55.

Taking Cetuximab as an example, the amino acid sequence of the heavy chain of the bifunctional molecule formed by the Fc-mutated EGFR antibody and IL15 is as shown in SEQ ID NO: 56; the amino acid sequence of the light chain is as shown in SEQ ID NO: 57.

Taking Atezolizumab, Avelumab, and Durvalumab as examples, the amino acid sequence of the heavy chain of the bifunctional molecule formed by the Fc-mutated PDL1 antibody and IL15 is as shown in SEQ ID NO: 58/60/62, respectively; the amino acid sequence of the light chain is as shown in SEQ ID NO: 59/61/63, respectively.

Taking Daratumumab as an example, the amino acid sequence of the heavy chain of the bifunctional molecule formed by an Fc-mutated CD38 antibody and IL15 is as shown in SEQ ID NO: 64; the amino acid sequence of the light chain is as shown in SEQ ID NO: 65.

Taking Veolizumab as an example, the amino acid sequence of the heavy chain of the bifunctional molecule formed by the Fc-mutated integrin $\alpha4\beta7$ antibody and IL15 is as shown in SEQ ID NO: 66; the amino acid sequence of the light chain is as shown in SEQ ID NO: 67.

Taking Alemtuzumab as an example, the amino acid sequence of the heavy chain of the bifunctional molecule formed by an Fc-mutated CD52 antibody and IL15 is as shown in SEQ ID NO: 68; the amino acid sequence of the light chain is as shown in SEQ ID NO: 69.

Taking Mogamuzumab as an example, the amino acid sequence of the heavy chain of the bifunctional molecule formed by an Fc-mutated CCR4 antibody and IL15 is as shown in SEQ ID NO: 70; the amino acid sequence of the light chain is as shown in SEQ ID NO: 71.

Taking Daclizumab, Basiliximab as an example, the amino acid sequence of the heavy chain of the bifunctional molecule formed by the Fc-mutated CD25 antibody and IL15 is as shown in SEQ ID NO: 72/74, respectively; the amino acid sequence of the light chain is as shown in SEQ ID NO: 73/75, respectively.

Taking Gemtuzumab as an example, the amino acid sequence of the heavy chain of the bifunctional molecule formed by an Fc-mutated CD33 antibody and IL15 is as shown in SEQ ID NO: 76; the amino acid sequence of the light chain is as shown in SEQ ID NO: 77.

Taking Ipilimumab as an example, the amino acid sequence of the heavy chain of the bifunctional molecule formed by an Fc-mutated CTLA4 antibody and IL15 is as shown in SEQ ID NO: 78; the amino acid sequence of the light chain is as shown in SEQ ID NO: 79.

Taking the antibody described in US Pat. No. 10,479,838 B2 as an example, the amino acid sequence of the heavy chain of the bifunctional molecule formed by an Fc-mutated CD40 antibody and IL15 is as shown in SEQ ID NO: 80; the amino acid sequence of the light chain is as shown in SEQ ID NO: 81.

Taking the antibody described in US20180280506A1 as an example, the amino acid sequence of the heavy chain of the bifunctional molecule formed by an Fc-mutated TIGIT antibody and IL15 is as shown in SEQ ID NO: 82; the amino acid sequence of the light chain is as shown in SEQ ID NO: 83.

Taking the antibody described in US Pat. No. 10,465,010 B2 as an example, the amino acid sequence of the heavy chain of the bifunctional molecule formed by an Fc-mutated GITR antibody and IL15 is as shown in SEQ ID NO: 84; the amino acid sequence of the light chain is as shown in SEQ ID NO: 85.

Taking the antibody described in US20190284277A1 as an example, the amino acid sequence of the heavy chain of the bifunctional molecule formed by an Fc-mutated LAG3 antibody and IL15 is as shown in SEQ ID NO: 86; the amino acid sequence of the light chain is as shown in SEQ ID NO: 87.

Taking the antibody described in US20070148739A1 as an example, the amino acid sequence of the heavy chain of the bifunctional molecule formed by an Fc-mutated CD138 antibody and IL15 is as shown in SEQ ID NO: 88; the amino acid sequence of the light chain is as shown in SEQ ID NO: 89.

**Embodiment 11 Other Fc Mutations That Reduce Binding of Antibodies to FcRn**

The method described above for reducing the *in vivo* toxicity of bifunctional molecules formed by antibodies and IL15 is achieved by Fc mutations, wherein Fc mutations decrease the binding of the bifunctional molecule to FcRn. In the embodiment, an Fc mutation method, i.e., the H3 10A/H435Q mutation, is used. However, other mutations that reduce the binding of the antibody to FcRn and achieve the same goal are also included in the present disclosure. These Fc mutations include, but are not limited to, the following: I253A mutation (Fc amino acid sequence as shown in SEQ ID NO : 90/104/117), S254A mutation (Fc amino acid sequence as shown in SEQ ID NO : 91/105/118), R255A mutation (Fc amino acid sequence as shown in SEQ ID NO : 92/106/119), K288A mutation (Fc amino acid sequence as shown in SEQ ID NO : 93/107/120), L309A mutation (Fc amino acid sequence as shown in SEQ ID NO : 94/121), H310A mutation (Fc amino acid sequence as shown in SEQ ID NO : 95/108/122), S415A mutation (Fc amino acid sequence as shown in SEQ ID NO : 96/109/123), H433A mutation (Fc amino acid sequence as shown in SEQ ID NO : 97/110/124), H435A mutation (Fc amino acid sequence as shown in SEQ ID NO : 98/111/125), H435R mutation (Fc amino acid sequence as shown in SEQ ID NO : 99/112/126), Y436A mutation (Fc amino acid sequence as shown in SEQ ID NO : 100/113/127), H310Q/H433N mutation (Fc amino acid sequence as shown in SEQ ID NO: 101/114/128), M252Y/T256Q mutation (Fc amino acid sequence as shown in SEQ ID NO: 102/115/129), M252F/T256D mutation (Fc amino acid sequence as shown in SEQ ID NO: 103/116/130), etc.

**Embodiment 12 Other Cytokines Besides IL15 to Form Bifunctional Molecules with Fe-Mutated Antibodies**

The above embodiments provides exemplary bifunctional molecules that are formed by IL-15 and Fc-mutated antibodies, indicating that the Fc mutation reduces binding to FcRn, thus leading to a significant reduction in the *in vivo* toxicity of these bifunctional molecules. In these bifunctional molecules, replacing IL-15 with other cytokines can achieve the same effect. These cytokines include, but are not limited to:

IL2 (amino acid sequence as shown in SEQ ID NO: 1) and variants thereof (amino acid sequences as shown in SEQ ID NO: 2/3/4);

IL12 (amino acid sequences as shown in SEQ ID NO: 5/7/9) and variants thereof (amino acid sequences as shown in SEQ ID NO: 131/132);

**Neo** 2/15 (amino acid sequence as shown in SEQ ID NO: 133);

IL7 (amino acid sequence as shown in SEQ ID NO: 134);

IL18 (amino acid sequence as shown in SEQ ID NO: 135);

IL21 (amino acid sequence as shown in SEQ ID NO: 136);

IL2-CD25 (amino acid sequences as shown in SEQ ID NO: 137/138/139);

IFNα (amino acid sequence as shown in SEQ ID NO: 140);

IFNα2b (amino acid sequence as shown in SEQ ID NO: 141) and a variant thereof (amino acid sequence as shown in SEQ ID NO: 142);

IFNγ (amino acid sequence as shown in SEQ ID NO: 143);

TNFα (amino acid sequence as shown in SEQ ID NO: 144);

GM-CSF (amino acid sequence as shown in SEQ ID NO: 145);

Flt3 (amino acid sequence as shown in SEQ ID NO: 146);

CCL21 (amino acid sequence as shown in SEQ ID NO: 147).

Although the present disclosure has been described in detail in the above embodiments, it is merely a part of the embodiments of the present disclosure, rather than all the embodiments. Those skilled in the art may further obtain other embodiments without any inventive step according to the above embodiments, and these embodiments all fall within the protection scope of the present disclosure.

## Claims

1.  A bifunctional molecule, comprising an Fc-mutated antibody and cytokine, wherein the Fc-mutated antibody and the cytokine are connected.

2.  The bifunctional molecule according to claim 1, wherein an Fc mutation in the Fc-mutated antibody is selected from: H310A/H435Q, I253A, S254A, R255A, K288A, L309A, H310A, S415A, H433A, H435A, H435R, Y436A, H310Q/H433N, M252Y/T256Q, and M252F/T256D.

3.  The bifunctional molecule according to claim 1, wherein the antibody is selected from: IgG1, IgG4, HER2, HER3, EGFR, PDL1, CD19, CD20, CD22, CD24, CD33, CD40, CD40L, CD73, CD276, VEGFR, TIGIT, TIM3, LAG3,CXCR3, CXCR5, CCR3, CCR4, CCR9, and PD1.

4.  The bifunctional molecule according to claim 1, wherein the cytokine is selected from: IL2 and mutants thereof, IL7, IL12 and mutants thereof, IL15, IL18, IL21, IL2-CD25, Neo 2/15, IFNα, IFNα2b and mutants thereof, IFNγ, TNFα, GM-CSF, FLt3, and CCL21.

5.  A method for amplifying the bifunctional molecule according to any one of claims 1-4, comprising:

    connecting the cytokine to the Fc-mutated antibody to form a fusion protein;
    constructing the fusion protein into an expression vector;
    transfecting the expression vector into cells, and
    performing purification.

6.  A therapeutic antibody drug, comprising the bifunctional molecule according to any one of claims 1-4.

7.  A use of the bifunctional molecule according to any one of claims 1-4 in the preparation of a drug.

8.  The use according to claim 7, wherein the drug is a therapeutic antibody drug.

FIG.1

FIG.2

## MC38 Xenograft Tumor Model in C57BL/6 Mice

FIG.3

## Proliferation of CTLL2 Cell

|  | IL15sc-Fcwt | IL15sc-Fcmu |
|---|---|---|
| EC50 | 3.455 | 3,516 |

FIG.4

## MC38 Xenograft Tumor Model in C57BL/6 Mice

FIG.5

## Proliferation of CTLL2 Cell

|  | IL2/h22F8mu | IL2/h22F8wt |
|---|---|---|
| EC50 | ~ 0.7419 | ~ 0.7485 |

FIG.6

FIG.7

|  | hu147wt-IL15sc | hu147mu-IL15sc |
|---|---|---|
| EC50 | 91.03 | 103.0 |

FIG.8

**MC38 Xenograft Tumor Model in C57BL/6 Mice**

Legend:
- Vehicle
- hu147mu-hIL15sc
- hu147wt-hIL15sc

Y-axis: Tumor Volume (mm3)
X-axis: Days

FIG.9

**Proliferation of CTLL2**

Legend:
- hu3E10wt-IL15sc
- hu3E10mu-IL15sc

Y-axis: OD450
X-axis: ng/ml

|  | hu3E10wt-IL15sc | hu3E10mu-IL15sc |
| --- | --- | --- |
| EC50 | 99.31 | 104.8 |

FIG.10

FIG.11

| | hu19H6wt-IL15sc | hu19H6mu-IL15sc |
|---|---|---|
| EC50 | 90.24 | 89.24 |

FIG.12

## MC38 Xenograft tumor model
## Tumor Volume (mm3) (SEM)

- ◆ Vehicle
- ----- hu19H6mu-hIL15sc 0.5mpk
- ——— hu19H6wt-hIL15sc 0.5mpk

FIG.13

## Proliferation of CTLL2

- ● hu609wt-15sc
- ■ hu609mu-15sc

|  | hu609wt-15sc | hu609mu-15sc |
|---|---|---|
| EC50 | 81.33 | 85.11 |

FIG.14

**MC38 Xenograft Tumor Model in 41BB Mice**

FIG.15

**Proliferation of CTLL2**

| | ch158wt-15sc | ch158mu-15sc |
|---|---|---|
| EC50 | 84.65 | 78.05 |

FIG.16

FIG.17

## INTERNATIONAL SEARCH REPORT

| International application No. |
|---|
| **PCT/CN2022/141443** |

| A. | CLASSIFICATION OF SUBJECT MATTER |
|---|---|

C07K19/00(2006.01)i; C12N15/85(2006.01)i; A61K38/20(2006.01)i; A61K39/00(2006.01)i

According to International Patent Classification (IPC) or to both national classification and IPC

| B. | FIELDS SEARCHED |
|---|---|

Minimum documentation searched (classification system followed by classification symbols)

Documentation searched other than minimum documentation to the extent that such documents are included in the fields searched

IPC: C07K, C12N, A61K

Electronic data base consulted during the international search (name of data base and, where practicable, search terms used)

CNABS; CNTXT; WPABS; WPABSC; ENTXTC; DWPI; VEN; CNKI; ISI; ELESEVER; NCBI; PUBMED; GOOGLE; GenBank; EMBL; STN; 万方, WANFANG; 中国专利生物序列检索系统数据库, China Patent Biological Sequence Retrieval System Database: Fc, 突变, 细胞因子, 融合蛋白, 抗体, mutat+, cytokine, fusion protein, antibody, IL-2, IL-12, IL-7, IL-15, SEQ ID NOS: 1-147

| C. | DOCUMENTS CONSIDERED TO BE RELEVANT |
|---|---|

| Category* | Citation of document, with indication, where appropriate, of the relevant passages | Relevant to claim No. |
|---|---|---|
| X | CN 102405230 A (MERCK PATENT GMBH) 04 April 2012 (2012-04-04) see description, paragraphs 0004-0114, and claims | 1, 2 (in part), 3-8 |
| Y | CN 102405230 A (MERCK PATENT GMBH) 04 April 2012 (2012-04-04) see description, paragraphs 0004-0114, and claims | 2 (in part) |
| X | EP 3766512 A1 (UNIV ZUERICH) 20 January 2021 (2021-01-20) see description, paragraphs 0013-0117, and claims | 1, 2 (in part), 3-8 |
| Y | EP 3766512 A1 (UNIV ZUERICH) 20 January 2021 (2021-01-20) see description, paragraphs 0013-0117, and claims | 1, 2 (in part), 3-8 |
| X | CN 114829385 A (OSE IMMUNOTHERAPEUTICS INC.) 29 July 2022 (2022-07-29) see claims | 1, 3-8 |
| Y | CN 114829385 A (OSE IMMUNOTHERAPEUTICS INC.) 29 July 2022 (2022-07-29) see claims | 2 |

☑ Further documents are listed in the continuation of Box C. ☑ See patent family annex.

| | |
|---|---|
| \* Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **29 June 2023** | **09 July 2023** |

| Name and mailing address of the ISA/CN | Authorized officer |
|---|---|
| **China National Intellectual Property Administration (ISA/ CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2022/141443** |

### C. DOCUMENTS CONSIDERED TO BE RELEVANT

| Category* | Citation of document, with indication, where appropriate, of the relevant passages | Relevant to claim No. |
| --- | --- | --- |
| Y | CN 105164157 A (F. HOFFMANN-LA ROCHE AG) 16 December 2015 (2015-12-16) see paragraphs [0022]-[0081], [0324]-[0402], and [0677]-[0936], and claims | 2 (in part) |
| A | CN 110214147 A (XENCOR INC.) 06 September 2019 (2019-09-06) see description, paragraphs 0161-0398, and claims | 1-8 |
| A | CN 113195523 A (XENCOR INC.) 30 July 2021 (2021-07-30) see claims | 1-8 |

Form PCT/ISA/210 (second sheet) (July 2022)

**INTERNATIONAL SEARCH REPORT**

| International application No. |
| --- |
| **PCT/CN2022/141443** |

**Box No. I        Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑ forming part of the international application as filed.

    b.  ☐ furnished subsequent to the international filing date for the purposes of international search (Rule 13*ter*.1(a)),

        ☐ accompanied by a statement to the effect that the sequence listing does not go beyond the disclosure in the international application as filed.

2.  ☐ With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

EP 4 603 513 A1

INTERNATIONAL SEARCH REPORT
Information on patent family members

International application No.

PCT/CN2022/141443

| Patent document cited in search report | | | Publication date (day/month/year) | Patent family member(s) | | | Publication date (day/month/year) |
|---|---|---|---|---|---|---|---|
| CN | 102405230 | A | 04 April 2012 | US | 2015274797 | A1 | 01 October 2015 |
| | | | | EP | 2421896 | A1 | 29 February 2012 |
| | | | | BRPI | 1016204 | A2 | 19 April 2016 |
| | | | | US | 2010272720 | A1 | 28 October 2010 |
| | | | | US | 8907066 | B2 | 09 December 2014 |
| | | | | WO | 2010121766 | A1 | 28 October 2010 |
| | | | | JP | 2012524528 | A | 18 October 2012 |
| | | | | JP | 5781501 | B2 | 24 September 2015 |
| | | | | CA | 2759333 | A1 | 28 October 2010 |
| | | | | IL | 215538 | A0 | 29 December 2011 |
| | | | | KR | 20120030383 | A | 28 March 2012 |
| | | | | EA | 201171259 | A1 | 30 May 2012 |
| | | | | SG | 175233 | A1 | 28 November 2011 |
| | | | | AU | 2010238858 | A1 | 08 December 2011 |
| | | | | MX | 2011011044 | A | 04 November 2011 |
| EP | 3766512 | A1 | 20 January 2021 | None | | | |
| CN | 114829385 | A | 29 July 2022 | WO | 2021122866 | A1 | 24 June 2021 |
| | | | | AU | 2020406083 | A1 | 16 June 2022 |
| | | | | TW | 202136287 | A | 01 October 2021 |
| CN | 105164157 | A | 16 December 2015 | WO | 2014177459 | A2 | 06 November 2014 |
| | | | | WO | 2014177459 | A3 | 24 December 2014 |
| | | | | ES | 2871383 | T3 | 28 October 2021 |
| | | | | EP | 2992010 | A2 | 09 March 2016 |
| | | | | EP | 2992010 | B1 | 24 March 2021 |
| | | | | CA | 2904805 | A1 | 06 November 2014 |
| | | | | RU | 2019108429 | A | 06 May 2019 |
| | | | | JP | 2020015723 | A | 30 January 2020 |
| | | | | JP | 6942162 | B2 | 29 September 2021 |
| | | | | US | 2016194389 | A1 | 07 July 2016 |
| | | | | HK | 1218761 | A1 | 10 March 2017 |
| | | | | MX | 2015015060 | A | 25 February 2016 |
| | | | | KR | 20160003818 | A | 11 January 2016 |
| | | | | KR | 102266819 | B1 | 18 June 2021 |
| | | | | US | 2020095310 | A1 | 26 March 2020 |
| | | | | JP | 2021138732 | A | 16 September 2021 |
| | | | | JP | 7240443 | B2 | 15 March 2023 |
| | | | | US | 2022324955 | A1 | 13 October 2022 |
| | | | | US | 2016068613 | A1 | 10 March 2016 |
| | | | | JP | 2016528166 | A | 15 September 2016 |
| | | | | JP | 6618893 | B2 | 11 December 2019 |
| | | | | PL | 2992010 | T3 | 23 August 2021 |
| | | | | MX | 2020003260 | A | 20 July 2020 |
| | | | | RU | 2015145719 | A | 05 June 2017 |
| | | | | RU | 2687043 | C2 | 06 May 2019 |
| | | | | EP | 3878866 | A1 | 15 September 2021 |
| | | | | US | 2020172607 | A1 | 04 June 2020 |
| CN | 110214147 | A | 06 September 2019 | WO | 2018071918 | A1 | 19 April 2018 |
| | | | | CO | 2019003945 | A2 | 30 April 2019 |
| | | | | MX | 2019004328 | A | 18 September 2019 |
| | | | | CO | 2019003943 | A2 | 30 April 2019 |

Form PCT/ISA/210 (patent family annex) (July 2022)

28

**INTERNATIONAL SEARCH REPORT**
Information on patent family members

International application No.

**PCT/CN2022/141443**

| Patent document cited in search report | Publication date (day/month/year) | Patent family member(s) | | Publication date (day/month/year) |
|---|---|---|---|---|
| | | MA | 46534 A | 21 August 2019 |
| | | JP | 2019533444 A | 21 November 2019 |
| | | JP | 7273453 B2 | 15 May 2023 |
| | | RU | 2019114175 A | 16 November 2020 |
| | | RU | 2019114175 A3 | 19 February 2021 |
| | | AU | 2022203820 A1 | 23 June 2022 |
| | | WO | 2018071919 A1 | 19 April 2018 |
| | | PE | 20191033 A1 | 05 August 2019 |
| | | CR | 20190227 A | 29 August 2019 |
| | | CL | 2019001000 A1 | 27 March 2020 |
| | | ZA | 201902383 B | 26 August 2020 |
| | | SG | 11201903302 UA | 30 May 2019 |
| | | CL | 2021003547 A1 | 20 September 2022 |
| | | AU | 2022203782 A1 | 23 June 2022 |
| | | PE | 20191034 A1 | 05 August 2019 |
| | | AU | 2017342560 A1 | 02 May 2019 |
| | | AU | 2017342560 B2 | 17 March 2022 |
| | | BR | 112019007288 A2 | 09 July 2019 |
| | | CL | 2022000097 A1 | 14 October 2022 |
| | | KR | 20190060821 A | 03 June 2019 |
| | | US | 2022195048 A1 | 23 June 2022 |
| | | EP | 3526241 A1 | 21 August 2019 |
| | | PH | 12019500782 A1 | 03 June 2019 |
| | | JP | 2022180450 A | 06 December 2022 |
| | | US | 2018118828 A1 | 03 May 2018 |
| | | US | 10550185 B2 | 04 February 2020 |
| | | SA | 519401562 B1 | 14 February 2023 |
| | | IL | 265998 A | 30 June 2019 |
| | | CA | 3040504 A1 | 19 April 2018 |
| | | KR | 20190065346 A | 11 June 2019 |
| | | MA | 46533 A | 21 August 2019 |
| | | MX | 2019004327 A | 14 October 2019 |
| | | US | 2018118805 A1 | 03 May 2018 |
| | | US | 10501543 B2 | 10 December 2019 |
| | | US | 2020040083 A1 | 06 February 2020 |
| | | US | 2020123259 A1 | 23 April 2020 |
| | | US | 11584794 B2 | 21 February 2023 |
| | | CL | 2019001001 A1 | 27 March 2020 |
| | | AU | 2017342559 A1 | 02 May 2019 |
| | | AU | 2017342559 B2 | 24 March 2022 |
| | | SG | 11201903304 YA | 30 May 2019 |
| | | ZA | 201902380 B | 26 August 2020 |
| | | RU | 2019114268 A | 16 November 2020 |
| | | RU | 2019114268 A3 | 19 February 2021 |
| | | PH | 12019500781 A1 | 03 June 2019 |
| | | JP | 2019533443 A | 21 November 2019 |
| | | JP | 7142630 B2 | 27 September 2022 |
| | | EP | 3526240 A1 | 21 August 2019 |
| | | IL | 265997 A | 30 June 2019 |
| | | CA | 3039930 A1 | 19 April 2018 |

Form PCT/ISA/210 (patent family annex) (July 2022)

## INTERNATIONAL SEARCH REPORT
### Information on patent family members

International application No.

**PCT/CN2022/141443**

| Patent document cited in search report | | | Publication date (day/month/year) | Patent family member(s) | | | Publication date (day/month/year) |
|---|---|---|---|---|---|---|---|
| | | | | BR | 112019007281 | A2 | 09 July 2019 |
| CN | 113195523 | A | 30 July 2021 | EP | 3861016 | A2 | 11 August 2021 |
| | | | | KR | 20210069641 | A | 11 June 2021 |
| | | | | US | 2022396605 | A1 | 15 December 2022 |
| | | | | US | 2023027540 | A1 | 26 January 2023 |
| | | | | US | 11655277 | B2 | 23 May 2023 |
| | | | | SG | 11202103192 | RA | 29 April 2021 |
| | | | | MA | 53822 | A | 11 August 2021 |
| | | | | US | 2020216509 | A1 | 09 July 2020 |
| | | | | US | 11358999 | B2 | 14 June 2022 |
| | | | | CA | 3115096 | A1 | 09 April 2020 |
| | | | | AU | 2019355971 | A1 | 06 May 2021 |
| | | | | IL | 281962 | A | 31 May 2021 |
| | | | | MX | 2021003765 | A | 15 July 2021 |
| | | | | WO | 2020072821 | A2 | 09 April 2020 |
| | | | | WO | 2020072821 | A9 | 22 May 2020 |
| | | | | WO | 2020072821 | A3 | 18 June 2020 |
| | | | | JP | 2022503959 | A | 12 January 2022 |

Form PCT/ISA/210 (patent family annex) (July 2022)

**EP 4 603 513 A1**

**REFERENCES CITED IN THE DESCRIPTION**

**Patent documents cited in the description**

- US 20200216509 A1
- US 20180118805 A1
- US 10479838 B2
- US 20180280506 A1
- US 10465010 B2
- US 20190284277 A1
- US 20070148739 A1